## Europäisches Patentamt

## European Patent Office

## Office européen des brevets

(19)

(11) Veröffentlichungsnummer : **0 129 899**
**B1**

(12) **EUROPÄISCHE PATENTSCHRIFT**

(45) Veröffentlichungstag der Patentschrift :
19.08.87

(21) Anmeldenummer : 84107277.0

(22) Anmeldetag : 25.06.84

(51) Int. Cl.⁴ : **C 07 C 5/25**, B 01 J 29/04,
**C 07 C 5/22**

(54) Verfahren zur Valenzisomerisierung von Olefinen.

(30) Priorität : 25.06.83 DE 3323022

(43) Veröffentlichungstag der Anmeldung :
02.01.85 Patentblatt 85/01

(45) Bekanntmachung des Hinweises auf die Patenterteilung : 19.08.87 Patentblatt 87/34

(84) Benannte Vertragsstaaten :
AT BE DE FR GB IT NL

(56) Entgegenhaltungen :
EP-A- 0 051 741
EP-A- 0 073 482
CHEMICAL ABSTRACTS, Band 99, Nr. 1, 4. Juli 1983,
Columbus, Ohio, USA; L. RIEKERT et al. "Sorption
and catalytic reaction in silicoaluminates and silicoborates of the pentasil class", Seite 456, Spalte 1,
Zusammenfassungsnr. 4941z
CHEMICAL ABSTRACTS, Band 102, Nr. 6, 11. Februar
1985, Columbus, Ohio, USA; B.S. RAO et al. "Isomerization of alkylaromatics over ferrosilicate zeolites",
Seite 105, Spalte 2, Zusammenfassungsnr. 47705y

(73) Patentinhaber : BASF Aktiengesellschaft
Carl-Bosch-Strasse 38
D-6700 Ludwigshafen (DE)

(72) Erfinder : Hoelderich, Wolfgang, Dr.
Mannheimer Strasse 18 c
D-6710 Frankenthal (DE)
Erfinder : Lindner, Alfred, Dr.
Ringstrasse 30
D-6712 Bobenheim-Roxheim (DE)
Erfinder : Mross, Wolf Dieter, Dr.
Anselm-Feuerbach-Strasse 21
D-6710 Frankenthal (DE)
Erfinder : Schwarzmann, Matthias, Dr.
Carl-Bosch-Strasse 54
D-6703 Limburgerhof (DE)
Erfinder : Volkamer, Klaus, Dr.
Heidelberger Ring 21
D-6710 Frankenthal (DE)
Erfinder : Wagner, Ulrich, Dr.
Knospstrasse 7
D-6703 Limburgerhof (DE)

**0 129 899**

## Beschreibung

Die vorliegende Erfindung betrifft ein Verfahren zur Valenzisomerisierung von Olefinen in Gegenwart von Katalysatoren.

Es ist bereits aus der US-PS 2 921 103 bekannt, 2-Butene bei 610 °C an einem Chromoxid-Aluminiumoxid-Katalysator unter Verwendung von Wasserdampf als Verdünnungsmittel zu 1-Buten zu isomerisieren. Bei diesem Verfahren werden jedoch trotz der Verwendung von Wasserdampf als Verdünnungsmittel nur Katalysatorlaufzeiten von 10 bis 100 Stunden erreicht. Wird das bekannte Verfahren ohne Zusatz von Wasserdampf durchgeführt, so werden gar nur Laufzeiten von bis zu 2 Stunden erhalten. Wegen der erforderlichen häufigen Katalysatorregenerierungen und wegen der erforderlichen hohen Wasserdampfverdünnung ist das Verfahren sehr arbeits- und energieaufwendig.

Es ist weiter aus Eesti NSV Tead. Akad. Tiom., Keem, Geol. 19(3), 206(1970) bekannt, 1-Hepten zu 2-Hepten und 3-Hepten und 1-Octen zu den Octenen in Gegenwart von Zeolithen des X-Typs zu isomerisieren. Auch bei diesem Verfahren werden nur unbefriedigende Katalysatorlaufzeiten und nicht zufriedenstellende Selektivitäten des Katalysators erhalten.

Die vorliegende Erfindung soll nun eine Verbesserung der Arbeitsweise und Wirtschaftlichkeit der bekannten Verfahren bewirken.

Es wurde nun ein vorteilhaftes Verfahren gefunden zur Valenzisomerisierung von Olefinen in Gegenwart eines Katalysators bei erhöhten Temperaturen, welches dadurch gekennzeichnet ist, daß man die Valenzisomerisierung der Olefine in Gegenwart von Zeolithen vom Pentasiltyp als Katalysatoren durchführt.

Nach dem neuen Verfahren werden auch ohne Wasserdampfzusatz hohe Laufzeiten des Isomerisierungskatalysators bei gleichbleibend hoher Selektivität erhalten. Es war überraschend, daß auch ohne Wasserdampfzusatz die Bildung von Crackprodukten und Skelettisomerisierungsprodukten vermieden werden und somit auf weitere Reinigungsstufen bei der Aufarbeitung des Produktgemisches verzichtet werden kann. Ein weiterer Vorteil der erfindungsgemäß zu verwendenden Katalysatoren liegt in ihrer Regenerierbarkeit. Aufgrund der relativ niedrigen Temperaturen, bei denen sich die Valenzisomerisierungen durchführen lassen, der erhöhten Katalysatorlaufzeiten und des geringen apparativen Aufwandes bei der Aufarbeitung läßt sich das erfindungsgemäße Verfahren in überaus wirtschaftlicher Weise mit niedrigem Energiebedarf durchführen.

Das neue Verfahren dient zur Valenzisomerisierung, d. h. zur Verschiebung der Doppelbindung unter Erhalt des Kohlenstoffgerüstes, von Olefinen mit im allgemeinen 4 bis 12, vorzugsweise 4 bis 10 Kohlenstoffatomen. Als erfindungsgemäß zu verwendende Olefine kommen beispielsweise geradkettige und verzweigte Alkene sowie Cycloalkene in Betracht. Geeignete Olefine sind beispielsweise 1-Buten, 2-Buten, 1-Penten, 2-Methyl-1-buten, 3-Methyl-1-buten, 2,3-Dimethyl-1-buten, 1-Hexen, 2-Methyl-1-penten, 4-Methyl-1-penten, Methylencyclohexen, 1-Octen, 1,3-Cyclooctadien, 1-Nonen, 1-Decen. Mit besonderem Vorteil wird das erfindungsgemäße Verfahren für die Valenzisomerisierung von Butenen oder Butengemischen, die noch Butane enthalten können, verwendet.

Als Katalysatoren werden für das erfindungsgemäße Verfahren Zeolithe vom Pentasiltyp verwendet. Diese Zeolithe können unterschiedliche chemische Zusammensetzung aufweisen. Es handelt sich hierbei um Alumino-, Boro-, Eisen-, Gallium-, Chrom-, Arsen- und Bismutsilikatzeolithe oder deren Gemische sowie um Alumino-, Boro-, Gallium- und Eisengermanatzeolithe oder deren Gemische. Die als Katalysatoren verwendeten Zeolithe vom Pentasil-Typ sind an sich bekannt. So wird die Herstellung eines Aluminosilikatzeoliths vom Pentasiltyp z. B. in EP-A-51 741 beschrieben. Weiter sind aus der EP-A-73 482 (Seite 1, Zeilen 26 bis 32) Zeolithe vom Pentasil-Typ bekannt, die Bor, Eisen, Arsen, Antimon, Vanadin oder Chrom enthalten. In der EP-A-73 482 ist weiter die Verwendung von Zeolithen vom Pentasil-Typ als Katalysatoren für die Umwandlung von Methanol zu Kohlenwasserstoffen beschrieben.

Für das erfindungsgemäße Verfahren eignen sich insbesondere Alumino- und Borosilikatzeolithe.

Die Aluminosilikatzeolithe werden beispielsweise in der Weise hergestellt, daß man eine Aluminiumverbindung, vorzugsweise ein Aluminiumhydroxid wie $Al(OH)_3$, oder ein Aluminiumsalz wie Aluminiumsulfat und eine Siliciumkomponente, zweckmäßig Siliciumdioxid in vorzugsweise hochdisperser Form, in zweckmäßig flüssigem Medium, gegebenenfalls mit Alkali- und/oder Erdalkalizusatz, bei erhöhten Temperaturen, im allgemeinen bei Temperaturen von 100 °C bis 200 °C, zweckmäßig unter autogenem Druck, miteinander umsetzt. Als flüssiges Medium kommen für die Zeolitherstellung beispielsweise wäßrige Alkalilauge, Alkohole wie Methanol, Ethanol, Propanol, Butanol, Ethylenglykol, 1,4-Butandiol gegebenenfalls in Mischung mit Wasser, Ether wie Ethylenglykolmonomethylether, Ethylenglykoldimethylether, Diethylenglykolmonomethylether, Diethylenglykoldimethylether gegebenenfalls in Mischung mit Wasser, vorzugsweise Amine wie 1,6-Hexandiamin, 1,3-Propandiamin, Triethylentetramin, zweckmäßig in Mischung mit Wasser, in Betracht.

Die Borosilikatzeolithe vom Pentasiltyp lassen sich beispielsweise entsprechend der vorstehend beschriebenen Herstellung der Aluminosilikatzeolithe herstellen, indem anstelle der Aluminiumverbindung eine Borverbindung, z. B. $HBO_3$, mit der Siliciumverbindung umgesetzt wird. Dabei werden vorzugsweise Reaktionstemperaturen 90 °C bis 170 °C angewendet.

Die erhaltenen Alumino- und Borosilikatzeolithe werden beispielsweise in der Weise zu dem

2

einzusetzenden Katalysator weiterverarbeitet, daß man sie nach ihrer Isolierung, Trocknung bei Temperaturen von im allgemeinen 80 °C bis 160 °C, vorzugsweise 100 °C bis 120 °C, und Calcination bei Temperaturen von zweckmäßig 450 °C bis 550 °C, vorzugsweise 490 °C bis 510 °C, zweckmäßig mit einem Bindemittel im Verhältnis von im allgemeinen 90 : 10 bis 40 : 60 Gew.% zu Strängen oder Tabletten verformt. Als Bindemittel eignen sich beispielsweise Aluminiumoxide, vorzugsweise Boehmit, Aluminosilikate, zweckmäßig in amorpher Form, mit einem $SiO_2/Al_2O_3$-Verhältnis von im allgemeinen 25 : 75 bis 95 : 5, vorzugsweise 25 : 75 bis 75 : 25, Siliciumdioxid, bevorzugt in hochdisperser Form, Gemische aus zweckmäßig hochdispersem Siliciumdioxid und hochdispersem Aluminiumoxid, zweckmäßig hochdisperses Siliciumdioxid und hochdispersem Aluminiumoxid, zweckmäßig hochdisperses Titandioxid und Ton. Nach der Verformung werden die Extrudate oder Preßlinge zweckmäßig getrocknet, im allgemeinen bei Temperaturen von 80 °C bis 160 °C, vorzugsweise 100 °C bis 120 °C, und danach calciniert, zweckmäßig bei Temperaturen von 450 °C bis 550 °C, vorzugsweise 490 °C bis 520 °C: Eine weitere Ausführungsform zur Weiterverarbeitung zu dem einzusetzenden Katalysator besteht darin, daß der isolierte Alumino- bzw. Borosilikatzeolith direkt nach der Trocknung verformt wird und erstmals nach der Verformung einer Calcination unterworfen wird.

Zur Erhöhung der Selektivität, der Standzeit und der Anzahl der möglichen Regenerierungen lassen sich unterschiedlichem Modifizierungen an den erfindungsgemäß zu verwendenden zeolithischen Katalysatoren vornehmen.

Eine Art der Modifizierung der Katalysatoren besteht darin, daß man den unverformten oder verformten Zeolithen mit Alkalimetallen wie Na, Erdalkalimetallen wie Ca, Mg, Erdmetallen wie B, Tl, Übergangsmetallen wie Mn, Fe, Mo, Cu, Zn und/oder Seltenen Erdmetallen wie La, Ce ionenaustauscht oder dotiert. Eine Ausführungsform für diese Modifizierung besteht beispielsweise darin, daß man den verformten Pentasilzeolithen in einem Steigrohr vorlegt und bei Raumtemperatur oder erhöhten Temperaturen, z. B. bei Temperaturen von 20 °C bis 120 °C, vorzugsweise 20 °C bis 100 °C, ein Salz der voranbeschriebenen Metalle, z. B. ein Halogenid oder ein Nitrat, darüberleitet. Ein derartiger Ionenaustausch kann z. B. an der Wasserstoff-, Ammonium- oder Alkaliform des Zeolithen vorgenommen werden. Eine weitere Ausführungsform für die Modifizierung besteht darin, daß man das zeolithische Material mit einer Verbindung der voranbeschriebenen Metalle, z. B. mit einem Halogenid, Nitrat und/oder Oxid, in flüssigem Medium, z. B. in wäßriger oder alkoholischer Lösung, imprägniert. Sowohl an einen Ionenaustausch als auch an eine Imprägnierung schließt sich zweckmäßig zumindest eine Trocknung und/oder Calcination an. Es kann vorteilhaft sein, bei den metalldotierten Zeolithen eine Nachbehandlung mit Wasserstoff anzuschließen.

Eine weitere Möglichkeit der Modifizierung besteht darin, daß man das unverformte oder verformte zeolithische Material einer Behandlung mit Säuren wie Salzsäure, Flußsäure, Phosphorsäure unterwirft. Eine besondere Ausführungsform für die Säurebehandlung besteht darin, daß man das zeolithische Material vor seiner Verformung bei erhöhter Temperatur mit Flußsäure, die im allgemeinen als 0,001 n bis 2 n, vorzugsweise 0,05 n bis 0,5 n Flußsäure eingesetzt wird, behandelt, beispielsweise durch Erhitzen unter Rückfluß über einen Zeitraum von im allgemeinen 0,5 bis 5, vorzugsweise 1 bis 3 Stunden. Nach Isolierung, z. B. durch Abfiltrieren und Auswaschen, des zeolithischen Materials wird dieses zweckmäßig, z. B. bei Temperaturen von 100 °C bis 160 °C, getrocknet und bei Temperaturen von im allgemeinen 450 °C bis 600 °C calciniert. Gemäß einer weiteren bevorzugten Ausführungsform für die Säurebehandlung wird das zeolithische Material nach seiner Verformung mit Bindemittel bei erhöhter Temperatur, zweckmäßig bei Temperaturen von 50 °C bis 90 °C, vorzugsweise 60 °C bis 80 °C, über einen Zeitraum von 0,5 bis 5, vorzugsweise 1 bis 3 Stunden mit Salzsäure, im allgemeinen mit 3 bis 25 Gew.%iger Salzsäure, vorzugsweise mit 12 bis 20 Gew.%iger Salzsäure, behandelt. Anschließend wird das zeolithische Material im allgemeinen ausgewaschen und zweckmäßig, z. B. bei Temperaturen von 100 °C bis 160 °C, getrocknet und bei Temperaturen von im allgemeinen 450 °C bis 600 °C calciniert.

Die erfindungsgemäß zu verwendenden Katalysatoren werden im allgemeinen in Form von Strängen, z. B. in Strängen von 2 mm bis 4 mm, Tabletten, z. B. als Tabletten mit 3 mm bis 5 mm Durchmesser, oder als Pulver, z. B. als Pulver mit Teilchengrößen von 0,1 bis 0,5 mm eingesetzt.

Es ist ein Vorteil der erfindungsgemäß zu verwendenden Katalysatoren, daß sie sich bei Nachlassen der Aktivität in einfacher Weise regenerieren lassen. Eine vorteilhafte Ausführungsform für die Regenerierung besteht darin, daß der Katalysator durch Abbrennen mit Sauerstoff enthaltenden Gasen, vorzugsweise Luft, regeneriert wird. Dabei kann es vorteilhaft sein, die Luft mit inerten Gasen, z. B. Stickstoff, zu verdünnen. Die Behandlung der zeolithischen Katalysatoren mit den Sauerstoff enthaltenden Gasen wird im allgemeinen bei Temperaturen von 300 °C bis 600 °C, vorzugsweise 400 °C bis 550 °C, insbesondere 480 °C bis 520 °C durchgeführt.

Die Valenzisomerisierung der Olefine wird im allgemeinen bei Temperaturen von 100 °C bis 500 °C durchgeführt. Bei der Isomerisierung von 1-Buten zu 2-Buten werden zweckmäßig Temperaturen von 100 °C bis 250 °C angewendet, während bei der Isomerizierung von 2-Buten zu 1-Buten im allgemeinen Temperaturen von 250 °C bis 500 °C angewendet werden. Die Belastung des Katalysators beträgt im allgemeinen 0,01 bis 20, vorzugsweise 0,05 bis 15 kg zu isomerisierendes Olefin je kg Katalysator pro Stunde. Die Valenzisomerisierung wird im allgemeinen bei Atmosphärendruck oder erhöhtem Druck, z. B. bis zu 100 bar, durchgeführt. Sie kann jedoch auch bei vermindertem Druck durchgeführt werden.

**0 129 899**

Folgende Beispiele veranschaulichen die Erfindung :

Beispiel 1

Herstellung von Katalysator A

Ein Bor-Zeolith des Pentasiltyps wurde in einer hydrothermalen Synthese aus 64 g $SiO_2$ (Aerosil 200), 12,2 g $H_3BO_3$, 800 g einer wäßrigen 1,6-Hexandiamin-Lösung (Mischung 50 : 50 Gew.%) bei 170 °C unter autogenem Druck in einem Rührautoklaven hergestellt. Nach Abfiltrieren und Auswaschen wurde das kristalline Reaktionsprodukt bei 110 °C 24 Stunden getrocknet und bei 500 °C 24 Stunden calciniert. Dieser Borosilikatzeolith setzte sich zusammen aus 94,2 Gew.% $SiO_2$ und 2,32 Gew.% $B_2O_3$.

Mit diesem Material wurden durch Verformen mit Boehmit im Gewichtsverhältnis 60 : 40 2 mm-Stränge hergestellt, die bei 110 °C 16 Stunden getrocknet und bei 500 °C 24 Stunden calciniert wurden.

Beispiel 2

Herstellung von Katalysator B

50 g des Borosilikatzeolithen aus Beispiel 1 wurden mit 140 ml 0,1 n Flußsäure unter Rückfluß 1 Stunde behandelt. Nach der Filtration und Auswaschen mit Wasser wurde bei 110 °C 16 Stunden getrocknet und bei 500 °C 5 Stunden calciniert. Dieses Produkt wurde mit Boehmit im Verhältnis 60 : 40 verstrangt, bei 110 °C 16 Stunden getrocknet und bei 500 °C 16 Stunden calciniert.

Beispiel 3

Herstellung von Katalysator C

Ein Aluminosilikatzeolith des Pentasiltyps wurde unter hydrothermalen Bedingungen bei autogenem Druck und 150 °C aus 65 g $SiO_2$ (Aerosil 200), 20,3 g $Al_2(SO_4)_3 \times 18\,H_2O$ in 1 kg einer wäßrigen 1,6-Hexandiamin-Lösung (Mischung 50 : 50 Gew.%) in einem Rührautoklaven synthetisiert. Nach Abfiltrieren und Auswaschen wurde das kristalline Reaktionsprodukt bei 110 °C 24 Stunden getrocknet und bei 500 °C 24 Stunden calciniert. Dieser Aluminosilikatzeolith enthielt 91,6 Gew.% $SiO_2$ und 4,6 Gew.% $Al_2O_3$.

Dieses Produkt wurde mit Boehmit im Gewichtsverhältnis 75 : 25 zu 2 mm-Strängen verformt und bei 110 °C 16 Stunden getrocknet und bei 500 °C 24 Stunden calciniert.

Beispiel 4

Herstellung von Katalysator D

50 g des Katalysators C wurden mit der Lösung von 9,39 g $Zn(NO_3)_2 \times 6\,H_2O$ in 20 g $H_2O$ getränkt. Nach der Tränkung wurde bei 130 °C 2 Stunden getrocknet und bei 540 °C 2 Stunden calciniert. Der Katalysator hatte einen Zn-Gehalt von 3,4 Gew.%.

Beispiel 5

Herstellung von Katalysator E

Katalysator E wurde erhalten, indem am Katalysator A ein Ionenaustausch mit einer Na-Verbindung bei Raumtemperatur vorgenommen wurde, wobei der Katalysator nach Auswaschen mit Wasser und 16-stündiger Trocknung bei 110 °C einen Na-Gehalt von 0,5 Gew.% aufwies.

Beispiele 6 bis 10

Die gemäß den Beispielen 1 bis 5 erhaltenen Katalysatoren A bis E wurden unter den in der Tabelle angegebenen Bedingungen für die Valenzisomerisierung von Butenen verwendet, wobei die in der Tabelle angegebenen Ergebnisse erhalten wurden. Bezüglich der Standzeit konnte z. B. beim Katalysator E bei der Umsetzung gemäß Beispiel 10 ohne Zugabe von Wasserdampf auch nach 220 Stunden Betriebszeit keine merkliche Desaktivierung festgestellt werden. Crackprodukte wie Propen oder Ethen sowie Skelettisomerisierungsprodukte wie Isobuten oder Dehydrierprodukte wie Butadien waren nicht nachweisbar.

(Siehe Tabelle Seite 5 f.)

4

# 0 129 899

Tabelle

| Beispiel | 6 | 7 | 8 | 9 | 10 |
|---|---|---|---|---|---|
| Einsatzstoff | Buten-1 | Buten-1 | Buten-1 | Buten-1 | trans-Buten-2 |
| Katalysator | A | B | C | D | E |
| Temperatur | 175°C | 200°C | 200°C | 250°C | 450°C |
| Belastung des Katalysators | $1\ h^{-1}$ | $1\ h^{-1}$ | $1\ h^{-1}$ | $1\ h^{-1}$ | $1\ h^{-1}$ |
| Produkte Gew.% | | | | | |
| trans-Buten-2 | 54,9 | 44,3 | 50,5 | 47,1 | 37,9 |
| cis-Buten-2 | 32,5 | 35,4 | 33,2 | 32,5 | 32,8 |
| Buten-1 | 12,0 | 19,8 | 15,7 | 19,7 | 26,7 |

## Patentansprüche

1. Verfahren zur Valenzisomerisierung von Olefinen in Gegenwart eines Katalysators bei erhöhten Temperaturen, dadurch gekennzeichnet, daß man die Valenzisomerisierung der Olefine in Gegenwart von Zeolithen vom Pentasiltyp als Katalysatoren durchführt.

2. Verfahren nach Anspruch 1, dadurch gekennzeichnet, daß man die Valenzisomerisierung der Olefine in Gegenwart von Boro- oder Aluminosilikatzeolithen vom Pentasiltyp durchführt.

3. Verfahren nach Anspruch 1 und 2, dadurch gekennzeichnet, daß man die Valenzisomerisierung der Olefine in Gegenwart von mit Alkalimetallen, Erdalkalimetallen und/oder Übergangsmetallen dotierten Zeolithen oder von durch Ionenaustausch mit Alkalimetallen, Erdalkalimetallen und/oder Übergangsmetallen erhältlichen Zeolithen durchführt.

4. Verfahren nach Anspruch 1 und 2, dadurch gekennzeichnet, daß man die Valenzisomerisierung der Olefine in Gegenwart eines durch Säurebehandlung modifizierten Zeolithen durchführt.

## Claims

1. A process for the valence isomerization of an olefin in the presence of a catalyst at elevated temperatures, wherein the valence isomerization of the olefin is carried out in the presence of a pentasil-type zeolite as the catalyst.

2. A process as claimed in claim 1, wherein the valence isomerization of the olefin is carried out in the presence of a borosilicate or aluminosilicate zeolite of the pentasil type.

3. A process as claimed in claims 1 and 2, wherein the valence isomerization of the olefin is carried out in the presence of a zeolite doped with an alkali metal, an alkaline earth metal and/or a transition metal, or of a zeolite obtainable by ion exchange with an alkali metal, an alkaline earth metal and/or a transition metal.

4. A process as claimed in claims 1 and 2, wherein the valence isomerization of the olefin is carried out in the presence of a zeolite modified by treatment with an acid.

## Revendications

1. Procédé de valence-isomérisation d'oléfines en présence d'un catalyseur et à température élevée, caractérisé en ce qu'on effectue la valence-isomérisation des oléfines en présence de zéolithes du type pentasil, servant de catalyseurs.

2. Procédé selon la revendication 1, caractérisé en ce qu'on effectue la valence-isomérisation des oléfines en présence de zéolithes de boro- ou d'aluminosilicate du type pentasil, servant de catalyseurs.

3. Procédé selon la revendication 1 ou 2, caractérisé en ce qu'on effectue la valence-isomérisation des oléfines en présence de zéolithes dopées par des métaux alcalins, des métaux alcalino-terreux et/ou des métaux de transition ou de zéolithes obtenues par échange d'ions avec des métaux alcalins, des métaux alcalino-terreux et/ou des métaux de transition.

4. Procédé selon la revendication 1 ou 2, caractérisé en ce qu'on effectue la valence-isomérisation des oléfines en présence d'une zéolithe modifiée par traitement par un acide.